(19) Europäisches Patentamt
European Patent Office
Office européen des brevets

(11) Publication number: **0 026 505**
**B1**

(12) **EUROPEAN PATENT SPECIFICATION**

(45) Date of publication of patent specification: **15.09.82**

(21) Application number: **80200781.5**

(22) Date of filing: **16.11.78**

(60) Publication number of the earlier application in accordance with Art. 76 EPC: **0002327**

(51) Int. Cl.[3]: **C 07 D 223/16,** C 07 D 491/04, C 07 D 405/06, C 07 D 409/06, A 61 K 31/55 //C07C47/575, C07C45/63, C07C45/71, C07C93/14

(54) **7,8-Substituted-1-phenyl-2,3,4,5-tetrahydro-1-H-3-benzazepines.**

(30) Priority: **17.11.77 US 852404**
**05.05.78 US 903325**

(43) Date of publication of application:
**08.04.81 Bulletin 81/14**

(45) Publication of the grant of the patent:
**15.09.82 Bulletin 82/37**

(84) Designated Contracting States:
**BE CH DE FR GB LU NL SE**

(56) References cited:
EP - A - 0 004 794
BE - A - 860 774
BE - A - 862 878
FR - A - 2 315 934
FR - A - 2 351 967
FR - A - 2 379 534
US - A - 4 104 379
US - A - 4 108 989

(73) Proprietor: **SMITHKLINE BECKMAN CORPORATION**
**1 Franklin Plaza**
**Philadelphia Pennsylvania 19101 (US)**

(72) Inventor: **Brush, Charles Kimball**
**2 Beverley Avenue**
**Malvern Pennsylvania 19355 (US)**
Inventor: **Weinstock, Joseph**
**1234 Pothouse Road**
**Phoenixville Pennsylvania 19406 (US)**

(74) Representative: **Waters, David Martin, Dr. et al,**
**P.O. Box 39**
**Welwyn Garden City, Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

7,8-Substituted-1-phenyl-2,3,4,5-tetrahydro-1-H-3-benzazepines

This invention comprises a new group of compounds which are useful as intermediates in preparing 1-phenyl-2,3,4,5-tetrahydro-1H-3-benzazepines having at least three substituents in the benz-ring of the nucleus one of which is a lower alkyl at the 6-position, said benzazepines being described and claimed in European Patent Application 78300633.1.

DESCRIPTION OF THE ART

Certain 1-phenyl-2,3,4,5-tetrahydro-1H-3-benzazepines have been described in Swiss Patent No. 555,831, including certain general methods of preparation of the 1-phenyl-2,3,4,5-tetrahydro-1H-3-benzazepine ring system. However this reference discloses no specific 6-lower alkyl substitution in the structures and is a broad generic disclosure.

DESCRIPTION OF THE INVENTION

According to the present invention there are provided compounds represented by the structural formula:

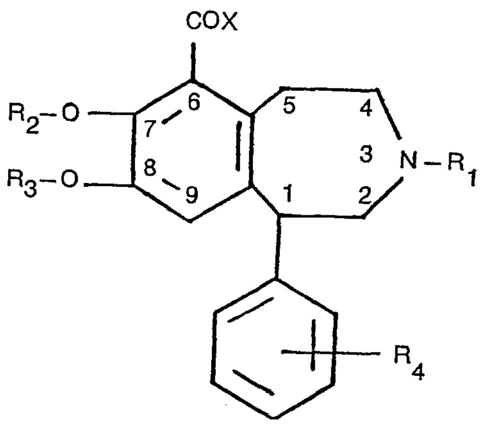

I

in which:

$R_1$ is hydrogen, benzyl, phenethyl, alkanoyl of from 1—5 carbons, such as formyl or acetyl, trifluoroacetyl, alkyl of 1—5 carbon atoms especially methyl, alkenyl of 3—5 carbon atoms such as allyl or dimethylallyl, hydroxyethyl, propargyl, furylmethyl, thienylmethyl or phenacyl;

$R_2$ and $R_3$ are each hydrogen, alkyl of 1—5 carbon atoms especially methyl, alkanoyl of 2—5 carbon atoms especially acetyl, benzyl or, when taken together, methylene;

$R_4$ is hydrogen or one or two substituents selected from trifluoromethyl, halo, e.g. chloro, bromo or fluoro, methyl, methoxy, acetoxy, methylthio or especially hydroxy, and

X is hydroxy, alkyl of 1—5 carbons or alkoxy of 1—5 carbons.

$R_2$ and $R_3$ are preferably methyl or benzyl. Prime blocking groups at positions 3 are trifluoroacetyl and formyl but others such as benzyl, phenethyl, lower alkanoyl, furoyl, thenoyl or phenacyl may be used.

The invention further provides acid addition and alkali metal salts of the compounds of formula I.

A subgeneric group of compounds within the above generic group are those of formula I in which:

$R_1$ is hydrogen, allyl, ethyl or methyl;

$R_2$ and $R_3$ are the same and are hydrogen, isobutyryl or acetyl; and

$R_4$ is hydrogen, *p*-hydroxy or *p*-acetoxy.

The compounds of this invention where X is hydroxy can be prepared by reacting an appropriate 6-lithium intermediate prepared from the corresponding 6-bromo congeners with carbon dioxide to form the 6-carboxy-1-phenyl-2,3,4,5-tetrahydro-1H-3-benzazepines. Subsequently the ester can be formed. Any chemical centers of the starting materials which may be reactive under the conditions for forming a 6-lithium benzazepine and reacting it with carbon dioxide should be protected as known to the art, for example by forming ether derivatives of hydroxy substituents or acylation of a reactive N-hydrogen center at the 3-position.

The 6-carboalkoxy compounds of formula I can be reacted with a lower alkyl lithium agent to give a 6-lower alkanoyl compound of formula I and as shown in formula II:—

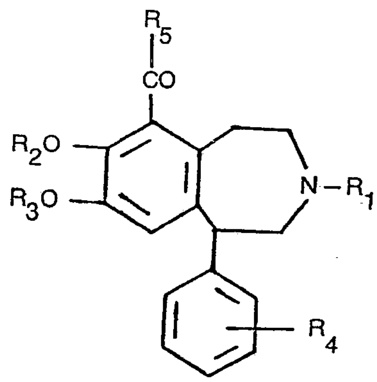

II

in which:

$R_5$ is alkyl containing 1—5 carbons; and

$R_1$—$R_4$ are as defined for formula I and if necessary, protected from the reaction conditions, as explained above and as illustrated in the Examples.

The following Examples illustrate the preparation of the compounds of this invention. The temperatures are Centigrade. Other variations of these Examples will be obvious to those skilled in the art.

Example 1

A. 7,8-Dimethoxy-1-phenyl-2,3,4,5-tetrahydro-1H-3-benzazepine (280 g, 0.75 mole) was dissolved in 1700 ml of acetic acid. Bromine (280 g, 1.75 mole) was added in a thin stream. This reaction was stirred for two hours. The precipitate, which formed after 1 hour, was collected and washed with ether. It was dissolved in boiling methanol and acetone was added to destroy the bromine excess. 6-Bromo-7,8-dimethoxy-1-phenyl-2,3,4,5-tetrahydro-1H-3-benzazepine hydrobromide was allowed to crystallize from the methanol and a second crop was obtained by adding ether to the mother liquor. Yield 298 g, 77% m.p. 236—238°. This bromination may be applied to any suitably O- and N-protected 7,8-dialkoxy or alkanoyloxybenzazepine having a free 6-position in which the substituted 1-phenyl ring is not more reactive to halogenation than is the 6-position of the nucleus.

B. Isovanillin (76.1 g, 0.5 m) was suspended in 750 ml of chloroform. Bromine (27.3 ml, 0.5 m) in 200 ml of chloroform was added at 0° slowly. Water was added to give the desired 2-bromo-3-hydroxy-4-methoxybenzaldehyde, m.p. 197—203°.

The aldehyde product (46.2 g, 0.2 mole) was dissolved in 300 ml of dry dimethylformamide, 69.1 g of potassium carbonate was added. 28.4 ml (0.30 mole) of dimethylsulfate was added at room temperature dropwise. After the addition the reaction was heated on the stream bath for 10 minutes. 29 ml of water was added dropwise and the reaction again heated for 5 minutes on the steam bath. The reaction was then poured into ice water and the precipitate collected, 2-bromo-3,4-dimethoxybenzaldehyde, m.p. 80—81.5°.

The dimethoxybenzaldehyde (10 g, 0.14 mole) was dissolved in 100 ml of ethanol and 5 g (0.132 mole) of sodium borohydride was added. The reaction was stirred for 1 hour. The reaction mixture was poured into water and extracted into methylene chloride to give the benzyl alcohol (m.p. 74—76.5°). This was converted to the benzyl chloride as a tan liquid, using benzene and conc. hydrochloric acid then to the benzyl cyanide, m.p. 48—55° using sodium cyanide in dimethylsulfoxide.

The benzyl cyanide (8.05 g, 0.315 mole) was dissolved in 80 ml of dry tetrahydrofuran and then added slowly to 80 ml of 1 *M* diborane in tetrahydrofuran at 5°. After refluxing for 2 hours, the mixture was cooled and 40 ml of methanol added carefully. After refluxing shortly and standing overnight the mixture was concentrated to give a tan oil. Dilute hydrochloric acid was added. The material was washed with ether, filtered and the filtrate made basic with 40% sodium hydroxide. After extracting with ether, washing, drying and evaporated the extracts the desired phenethylamine was obtained as a viscous, light yellow oil.

The phenethylamine (0.12 mole) is heated to 115° in an oil bath. Styrene oxide (14.4 g 0.12 mole) is added and the reaction heated for 1 hour. After cooling to 30°, 2:1 petroleum ether/acetone is added to give (N-[(2-hydroxy-2-phenylethyl)]-N-[2-(2′-bromo-3′-4′-dimethoxyphenyl)-ethyl]-amine.

The hydroxyphenethylamine (0.0445 mole) is dissolved in 60 ml of trifluoroacetic acid and 4.05 ml of concentrated sulfuric acid is added. The reaction is refluxed for 2 hours. After cooling most of the trifluoroacetic acid is stripped off and the residue is pured into water. It is made basic with 10% sodium hydroxide and extracted with ether twice. The ether is dried and evaporated to give 6-bromo-7,8-dimethoxy-1-phenyl-2,3,4,5-tetrahydro-1H-3-benzazepine.

Using this general procedure with variously substituted styrene oxides having one or more methyl, methoxy, methylthio, trifluoromethyl groups gives the corresponding 6-bromo intermediates not readily prepared by direct bromination which are used for preparing the 6-carboxy, carbalkoxy and alkanoyl compounds of this invention by the methods described hereafter.

The 6-bromo-7,8-dimethoxy compounds (100 g) in a large excess of ethyl formate is heated at reflux for 10 hours. Evaporation in vacuo and purification by fractional recrystallization gives the 3- or N-formyl derivative.

Example 2

A. A mixture of 25 g (0.068 mole) of 6-bromo-7-8-dimethoxy-1-phenyl]-2,3,4,5-tetrahydro-1H-3-benzazepine in a minimum amount of toluene diluted with ethyl ether under dry, degassed conditions was reacted with 100 ml (0.24 mole) of n-butyl lithium in 100 ml of ethyl ether at −78°.

After a few minutes, the mixture was poured onto a slurry of dry ice (carbon dioxide). The ether slurry was extracted with water. The aqueous extracts were extracted with ether, then acidified with dilute hydrochloric acid and warmed on the steam bath briefly. The acid layer was extraced with ether then stripped to leave a solid produce, 6-carboxy-7,8-dimethoxy-1-phenyl-2,3,4,5-tetrahydro-1H-3-benzazepine hydrochloride, m.p. 225—226° (dec.). More product, m.p. 230—235°C, was obtained from the mother liquor by use of sodium bicarbonate, ethyl acetate extraction and acidification with hydrochloric acid.

The 6-carboxy compound (3.0 g) was dissolved in methylene chloride and reacted with an excess of trifluoroacetic anhydride. After stirring for one hour, the mixture was cooled to 0° and an excess of methanol added. The volatiles were stripped. The residue was taken up in methylene chloride, washed with hydrochloric acid, then sodium bicarbonate solution. The organic layer was dried and evaporated to give the 3-trifluoroacetyl compound.

A mixture of 9.5 g (0.21 mole) of 6-carbomethoxy-7,8-dimethoxy-1-phenyl-3-trifluoroacetyl-2,3,4,5-tetrahydro-1H-3-benzazepine in dry toluene was reacted with 39 ml of methyl lithium in 100 ml of dry ethyl ether at 0° for one hour after dropwise addition.

The reaction was worked up by adding an excess of 10% hydrochloric acid and allowing it to stand at room temperature overnight. The non-organic layer and precipitate were collected, made basic with alkali and extracted by ethyl acetate. The toluene reaction mixture was taken through the salt isolation. The combined product as the base was dissolved in ether, made acid with ethereal hydrogne chloride to form the hdyrochloride salt and purified by recrystallization from methanol-ethyl acetate to give 6-acetyl-7,8-dimethoxy-1-phenyl-2,3,4,5-tetrahydro-1H-3-benzazepine hydrochloride, m.p. 235—236°.

B. A mixture of 4 g (0.1 mole) of 6-carboxy-7,8-dihydroxy-3-trifluoroacetyl-1-phenyl-2,3,4,5-tetrahydro-1H-3-benzazepine, prepared by boron tribromide treatment of the 7,8-dimethoxy congener, in 50 ml of dry dimethylformamide was reacted with 0.35 ml of benzyl chloride and 8.4 g of potassium carbonate at 160—175°. The mixture was poured onto ice and taken through ether to give 93% of the desired dibenzyl ether benzyl ester.

This material, 6.2 g, was reacted with methyl lithium in ethyl ether at 0° as described above to give the 6-acetyl-7,8-dibenzyloxy-1-phenyl-2,3,4,5-tetrahydro-1H-3-benzazepine, m.p. 229—233°.

Example 3

A mixture of 10 g (0.0255 mole) of 6-bromo-7,8-dimethoxy-1-(*p*-methoxyphenyl)-2,3,4,5-tetrahydro-1H-3-benzazepine and 42 ml of *n*-butyl lithium was reacted to give the 6-lithium derivative which is reacted with dry ice in excess as described above to give 6-carboxy-7,8-dimethoxy-1-(*p*-methoxyphenyl)-2,3,4,5-tetrahydro-1H-3-benzazepine hydrochloride, m.p. 242—245° (dec.).

This compound is esterified in *n*-butanol-hydrogen chloride to give the 6-carbobutoxy compound.

## Claims

1. A compound of the formula:

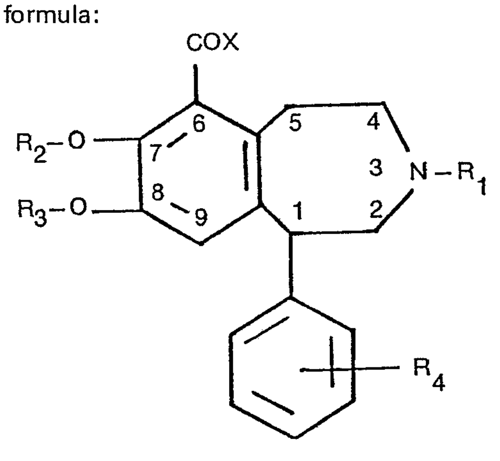

I

in which:

$R_1$ is hydrogen, benzyl, phenethyl, alkanoyl of 1—5 carbons, trifluoroacetyl, alkyl of 1—5

carbons, alkenyl of 3—5 carbons, hydroxyethyl, propargyl, furylmethyl, thienylmethyl or phenacyl;

$R_2$ and $R_3$ are each hydrogen, alkyl of 1—5 carbons alkanoyl of 2—5 carbons, benzyl or, when taken together methylene;

$R_4$ is hydrogen or one or two substituents selected from trifluoromethyl, halo, methyl, methoxy, acetoxy, hydroxy or methylthio; and

X is hydroxy, alkyl of 1—5 carbons or alkoxy of 1—5 carbon atoms;

or an acid addition or alkali metal salt thereof.

2. The compound of claim 1 which:

$R_1$ is hydrogen or methyl;

$R_2$ and $R_3$ are hydrogen;

$R_4$ is hydrogen, *p*-acetoxy or *p*-hydroxy; and

X is hydroxy or alkoxy of 1—5 carbons.

3. The compound of claim 1 in which X is hydroxy or alkoxy of 1—5 carbons.

4. The compound of claims 1, 2 and 3 in which $R_2$ and $R_3$ are hydrogen or acetyl and $R_1$ is hydrogen or methyl.

**Revendications**

1. Composé de formule:

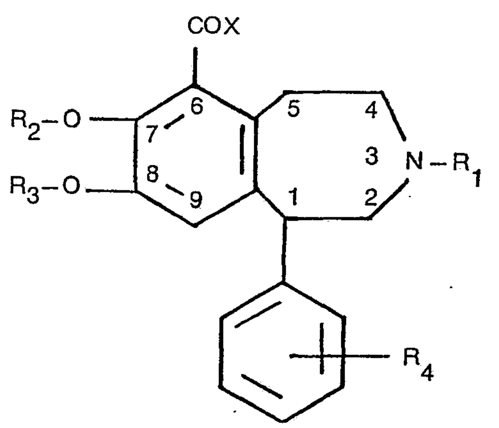

I

dans laquelle:

$R_1$ représente un atome d'hydrogène, un groupe benzyle, phénéthyle, alcanoyle ayant de 1 à 5 atomes de carbone, trifluoroacétyle, alcoyle ayant de 1 à 5 atomes de carbone, alcényle ayant de 3 à 5 atomes de carbone, hydroxyéthyle, propargyle, furylméthyle, thiénylméthyle ou phénacyle;

$R_2$ et $R_3$ représentent indépendamment l'un de l'autre un atome d'hydrogène, un groupe alcoyle ayant de 1 à 5 atomes de carbone, alcanoyle ayant de 2 à 5 atomes de carbone, benzyle ou forment ensemble un groupe méthylène;

$R_4$ représente un atome d'hydrogène ou un ou deux substituants choisis parmi les groupes trifluorométhyle, halo, méthyle, méthoxy, acétoxy, hydroxy ou méthylthio; et

X représente un groupe hydroxy, alcoyle ayant de 1 à 5 atomes de carbone ou alcoxy ayant de 1 à 5 atomes de carbone;

ou le sel d'addition d'acide ou de métal alcalin de ce composé.

2. Composé suivant la revendication 1 dans lequel:

$R_1$ représente un atome d'hydrogène ou un groupe méthyle;

$R_2$ et $R_3$ représentent un atome d'hydrogène;

$R_4$ représente un atome d'hydrogène, un groupe *p*-acétoxy ou *p*-hydroxy; et

X représente un groupe hydroxy ou alcoxy ayant de 1 à 5 atomes de carbone.

3. Composé suivant la revendication 1, dans lequel X représente un groupe hydroxy ou alcoxy ayant de 1 à 5 atomes de carbone.

4. Composé suivant les revendications 1, 2 et 3, dans lequel $R_2$ et $R_3$ représentent un atome d'hydrogène ou un groupe acétyle et $R_1$ représente un atome d'hydrogène ou un groupe méthyle.

**Patentansprüche**

1. Verbindung der Formel

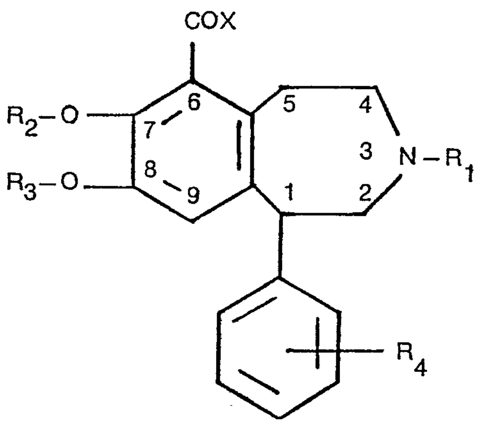

I

in der

$R_1$ ein Wasserstoffatom, eine Benzyl-, Phenäthyl-, $C_{1-5}$-Alkyl-, $C_{3-5}$-Alkenyl-, Hydroxyäthyl-, Propargyl-, Furylmethyl-, Thienylmethyl- oder Phenacylgruppe bedeutet;

$R_2$ und $R_3$ je ein Wasserstoffatom, eine $C_{1-5}$-Alkyl-, $C_{2-5}$-Alkanoyl- oder Benzylgruppe oder $R_2$ und $R_3$ zusammen eine Methylengruppe bedeuten;

$R_4$ ein Wasserstoffatom oder ein oder zwei Substituenten aus der Gruppe Trifluormethyl, Halogen, Methyl, Methoxy, Acetoxy, Hydroxy oder Methylthio bedeutet; und

X ein Wasserstoffatom, eine $C_{1-5}$-Alkyl- oder $C_{1-5}$-Alkoxygruppe bedeutet;

oder ihr Säureadditions- oder Alkalimetallsalz.

2. Verbindung nach Anspruch 1, in der

$R_1$ ein Wasserstoffatom oder eine Methylgruppe,
$R_2$ und $R_3$ ein Wasserstoffatom,
$R_4$ ein Wasserstoffatom, eine Acetoxy- oder p-Hydroxygruppe und
X ein Wasserstoffatom- oder eine $C_{1-5}$-Alkoxygruppe bedeuten.

3. Verbindung nach Anspruch 1, in der X eine Hydroxyl- oder $C_{1-5}$-Alkoxygruppe bedeutet.

4. Verbindung nach Anspruch 1, 2 und 3, in der $R_2$ und $R_3$ ein Wasserstoffatom oder eine Acetylgruppe und $R_1$ ein Wasserstoffatom oder eine Methylgruppe bedeuten.